Europäisches Patentamt

European Patent Office

Office européen des brevets

(19)

(11) EP 0 747 728 B1

(12) EUROPEAN PATENT SPECIFICATION

(45) Date of publication and mention
of the grant of the patent:
02.01.2003 Bulletin 2003/01

(51) Int Cl.⁷: G01T 1/164

(21) Application number: 96302370.0

(22) Date of filing: 03.04.1996

(54) **Improved gamma camera imaging system**

Verbessertes Gammakamera-Abbildungssystem

Système amélioré avec d'imagerie à caméra de gamma

(84) Designated Contracting States:
**DE FR GB**

(30) Priority: **11.05.1995 US 439111**
**11.05.1995 US 438834**
**11.05.1995 US 439134**
**11.05.1995 US 439222**

(43) Date of publication of application:
**11.12.1996 Bulletin 1996/50**

(60) Divisional application:
**01129807.2 / 1 205 768**
**01129818.9 / 1 205 769**
**01129772.8 / 1 205 767**

(73) Proprietor: **Koninklijke Philips Electronics N.V.**
**5621 BA Eindhoven (NL)**

(72) Inventors:
 • **Jones, Steven M.**
 **Pleasanton, California 94566 (US)**
 • **Lang, Thomas F.**
 **Fremont, California 94555 (US)**
 • **Janicki, Michael J.**
 **San Jose, California 95124 (US)**
 • **Liebig, John R.**
 **San Jose, California 92126 (US)**
 • **Brown, J. Keenan**
 **San Jose, California 95125 (US)**
 • **Wang, Xiaohan**
 **Alameda, California 94502 (US)**

(74) Representative: **Wombwell, Francis et al**
**Potts, Kerr & Co.**
**15, Hamilton Square**
**Birkenhead Merseyside CH41 6BR (GB)**

(56) References cited:
**US-A- 5 379 333** **US-A- 5 400 378**

 • **TAN P ET AL: "A SCANNING LINE SOURCE FOR SIMULTANEOUS EMISSION AND TRANSMISSION MEASUREMENTS IN SPECT" JOURNAL OF NUCLEAR MEDICINE, vol. 34, no. 10, October 1993, pages 1752-1760, XP000601777**

**Description**

BACKGROUND OF THE INVENTION

(1) Field of the Invention

The present invention relates to the field of nuclear medicine. Particularly, the present invention relates to an improved gamma camera imaging system.

(2) Background of the Invention

Non-uniform photon attenuation is an important factor that affects the quantitative accuracy of images collected using Single Photon Emission Computerized Tomography (SPECT) camera systems and can decrease the sensitivity of these systems for lesion detection. Non-uniform photon attenuation distorts images by interfering with and partially absorbing the radiation emitted from an organ containing a radio-pharmaceutical. Photon attenuation within SPECT systems tends to degrade images by also introducing image artifacts and other distortions that can result in false positive detection or the undetection of lesions. The effects of photon attenuation are especially complex in cardiac studies as a result of nonuniform attenuation attributed to the thorax.

Transmission computed tomography (TCT) can be used as a method for generating a nonuniform attenuation correction distribution. The transmission image data is gathered using a known source (e.g., line, sheet, or flood) of radiation. In transmission scanning, the source of radiation is directed toward the associated scintillation detector through the object of interest or patient. If the radiation field is significantly larger than the patient, the radiation source is allowed to directly radiate the detector, causing a high count rate in the scintillation detector. Those parts of the detector that become directly radiated are called unobstructed portions of the detector. It is not advantageous to allow large unobstructed detector areas, because the resultant increase in count rate can lead to image degradation and in some cases the event detection electronics and processes can become overloaded (e.g., due to pulse pile-up) and temporarily terminate operation. These high count rates tend to reduce the imaging performance of the imaging system by loading down the signal detection and processing circuitry of the gamma camera.

In the prior art, fixed "bow tie" filters were used in conjunction with line sources for transmission operations. The bow tie filter is used to reduce the count rate over detector areas that are unobstructed by the object being scanned. However, the use of a fixed bow tie filter is not advantageous because variably sized patients create differently sized unobstructed areas on the detector. A fixed bow tie filter is not advantageous because it will not account for patient variance and further requires effort to exchange in order to install new filters.

Also, during ECT scanning, different portions of the object become within the detector's field of view at different rotation angles, therefore different parts of the detector become unobstructed from the line source at different ECT angles. It would be advantageous, then, to provide a system to account for variable sized patients and also to account for different unobstructed portions of a detector for given ECT rotation angles during transmission sessions.

In addition, in certain SPECT imaging applications, such as with cardiac imaging, it is desirable to image the heart (or other organ) with high resolution image matrix (e.g., having small pixel sizes) because of the small size of the organ. In order to achieve sufficiently small pixel sizes, a small field of view detector is typically used. For example, detectors having a physical field of view of 16"x16", 15"x15" and 13"x13" are used.

However, in order to correct for nonuniform attenuation, as discussed above, a transmission map of the body is acquired. Imaging the whole body requires the full field of view of a large detector (e.g., 20"x15"). Therefore, there are problems when performing both SPECT and transmission imaging with a SFOV (small field of view) detector or performing both with a LFOV (large field of view) detector. If the large field of view is used for both transmission and emission, the pixels become too large and image resolution is lost for the emission data.

One solution to this problem is to acquire both the emission and the transmission data using the same small field of view. However, this approach is problematic because in order to determine a proper transmission map to correct the emission data, transmission information regarding the entire body is required -- not just the portion of the body imaged in the small field of view. The transmission data becomes truncated. When the body is truncated, artifacts are introduced which must be addressed by complex and cumbersome correction algorithms, which may in themselves be subject to error. In this approach, the transmission data is corrected to account for the fact that the body is larger than the field of view of the detector being used to acquire the transmission image. This correction, called truncation correction, assumes a known shape for the contour of the body and uses this assumption in conjunction with a special algorithm to calculate what the transmission data should have been in those parts of the body outside of the detector's field of view. In effect, this approach attempts to reconstruct a transmission image with an incomplete or truncated set of transmission data.

The above approach has several drawbacks. The data used to generate the transmission map is incomplete and the precision to which the body contour information needs be known is not fully understood or appreciated. This typically can lead to image degradation. In addition, this type of transmission truncation correction requires that the patient be positioned with extreme care, resulting in increased set-up time. Also, the susceptibility of this transmission correction approach to high degrees of image noise is not fully understood or appreciated. Therefore, it would be desirable to gather transmission information that is not truncated in order to improve the nonuniform attenuation corrections factors that are used to correct the emission data.

Typically, no attenuation correction is employed in myocardial SPECT, making it difficult for the physician to accurately diagnose lesions in cases where the patient is large-breasted, or where the diaphragm lies over the heart. Some investigators have used attenuation maps which assume uniform density inside the body contour; however, the uniform maps have tended to introduce artifacts into the reconstruction images. To correct for non-uniform attenuation due to the breasts, lungs and diaphragm, some investigators and manufacturers employ radionuclide transmission sources mounted opposite the gamma camera detector to directly measure the attenuation factors for each patient. These factors may then be used in conjunction with an iterative reconstruction process to correct for non-uniform attenuation.

A transmission study may be performed simultaneously with an emission study. If performed separately from the SPECT emission study, the collection of the transmission data requires additional data acquisition time and the collection of the transmission and emission data is susceptible to misregistration effects due to patient (e.g., "object") movement between the data gathering sessions. Among other advantages, simultaneous acquisition of both transmission and emission data reduces the effects of misregistration. In the case of a gamma camera with a single scintillation detector, it is known to use a sliding window or "band" associated with the field of view of the scintillation detector to move in conjunction with the line source to aid in allowing the gamma camera to differentiate between detected transmission photons and emission photons. For example, reference is made to an article entitled, "A Scanning Line Source for Simultaneous Emission and Transmission Measurements in SPECT," by Patrick Tan, et al., published in the Volume 34, No 10, of the Journal of Nuclear Medicine, in October 1993, which is incorporated by reference herein. This reference discloses use of a single scanning line source with a single moving detection band.

However, this solution offered by Tan et al. does not adequately account for "side scatter" or "crosstalk" in emission studies involving two scintillation detectors (e.g., within dual head detector systems). Crosstalk in emission studies involves transmission photons scattering off of an object (e.g., cause by Compton scatter) being studied and improperly detected by a scintillation detector as emission photons. In a dual detector gamma camera, transmission photons emitted from a line source that is associated with a given detector may be improperly detected (e.g., after scatter) as proper emission photons by the other detector. This is the case because the scattered photon loses energy as a function of the scatter angle and changes energy level. Cross-talk may also occur from emission photons that scatter off the object. In dual head gamma cameras, the effects of cross-talk are dealt with by performing a post processing operation on the detected data. This post processing operation is time consuming and not entirely accurate. Therefore, it would be advantageous to eliminate the need for such post processing step by eliminating the detection of the cross-scattered photons.

The use of a single sliding detection window associated with a single gamma camera detector does little to prevent cross-talk in a dual detector system. What is needed is a system that is operable within a dual detector camera system that effectively eliminates the improper detection of emission photons due to crosstalk.

In addition, there is a dual detector gamma camera system that employs tracking zoom regions (e.g., window regions) that are designed to track the motion of an object of interest during ECT motion. Within this system, the zoomed regions of the detector change as the detector rotates through ECT motion about the object. Reference is made to U.S. Patent No. 5,304,806, entitled, "Apparatus and Method for Automatic Tracking of a Zoomed Scan Area in a Medical Camera System," issued April 19, 1994, and assigned to the assignee of the present invention, which discusses tracking zoom regions.

Whether the transmission data is collected simultaneously with the collection of the emission data or not, the patient is generally exposed to additional radiation in order to collect the transmission data. Depending on the size and shape of the patient, different amounts of transmission radiation are required in order to collect the minimum required amounts of transmission data. Systems overexpose the patient with transmission radiation in order to obtain the minimum required amount without regard to the shape or size of the patient. What is needed is a system that can effectively minimize the exposure period of the patient to radiation utilized in collection of the transmission data by considering the particular size and shape contributions of the patient.

A variable radiation dose application is known from, e.g., US-5379333.

SUMMARY OF THE INVENTION

A scan speed procedure and method is described wherein a minimum radiation exposure period is determined on an object by object basis for a transmission study. Two transmission scan phases are performed including a prescan followed by a second transmission scan phase. The first scan consists of a transmission prescan performed using a radiation source over the object. This prescan is of a rapid and predetermined duration (Tp). A resultant count density associated with the object is then generated and examined. The portion having the smallest count density (Cm) is determined and a value (Co) representing the minimum required number of counts for transmission study is given. From the above, a transmission period (Ts) is determined by the system and the second transmission phase (a multi-pass scan phase) is performed for the duration Ts. The computed duration Ts represents the minimum exposure duration required to collect transmission data to ensure that the count density distribution associated with the object will contain at least Cm count density over each portion of the object.

## BRIEF DESCRIPTION OF THE DRAWINGS

Figure 1 is an illustration of the dual head detector system of the present invention with the line source assemblies configured for scanning and the detectors at 90 degrees configuration.

Figure 2 illustrates the dual head detector system of the present invention with the line source assemblies withdrawn for storage and the detectors at 180 degrees configuration.

Figure 3 is an illustration of a cross sectional view of a line source assembly of one embodiment of the present invention.

Figure 4 illustrates a block diagram of a general purpose computer system coupled to the detector system electronics of the present invention.

Figure 5 is an illustration of an exemplary configuration for performing a speed scan operation within the present invention.

Figure 6 is a flow diagram illustrating process steps for performing a speed scan operation within the present invention.

Figure 7 illustrates a count density relationship for a given patient profile within the scan speed operation of the present invention.

## DETAILED DESCRIPTION OF THE INVENTION

In the following detailed description of the present invention, numerous specific details are set forth in order to provide a thorough understanding of the present invention. However, it will be obvious to one skilled in the art that the present invention may be practiced without these specific details. In other instances well known methods, procedures, components, and circuits have not been described in detail as not to unnecessarily obscure aspects of the present invention. Unless specifically stated otherwise as apparent from the following discussions, it is appreciated that throughout the present invention, discussions relating to functions of the computer system of the present invention utilizing terms such as "processing" or "computing" or "calculating" or "determining" or "displaying" or the like, refer to the action of a computer system, or similar electronic computing device, that is executing a program to manipulate and transform data represented as physical (electronic) quantities within the computer system's registers and memories into other data similarly represented as physical quantities within the computer system memories or registers.

## I. NONUNIFORM ATTENUATION CORRECTION SYSTEM

Embodiments of the present invention relate to the collection, generation and usage of nonuniform attenuation correction factors used to improve gamma camera imaging. Since each patient that is imaged by a nuclear medicine gamma camera is different (e.g., differently shaped with different sizes, etc.) the tissue and bone structure that surround an organ of interest is different for each patient. This surrounding tissue and bone structure attenuates the radiation emitted from a radiopharmaceutical distributed within the imaged organ. The attenuation of the radiation is nonuniform because the attenuation coefficients of the different tissues and bone are different. Radiation attenuation non-uniformly reduces the count density in the image. This attenuation can lead to falsely identifying an artifact when in fact healthy tissue is imaged and vice-versa. If an artifact is improperly diagnosed as a lesion, this can lead to invasive measures which are painful and potentially dangerous (e.g., involves a health risk) for the patient.

Nonuniform attenuation caused by the body can be compensated for if the attenuation map of the body is known. Transmission scanning allows a gamma camera and a processing computer system to generate a nonuniform attenuation map of a particular object. This nonuniform attenuation map can be obtained for each rotation angle of a scintillation detector so that a reconstruction algorithm can use the attenuation map for each angle. Generally, during transmission scanning, a source of known radiation is emitted through the patent to be scanned and then the radiation is detected by a scintillation detector. By measuring the intensity of the radiation emitted from the source, and by measuring the intensity of radiation emitted through the object at different ECT angles, the gamma camera's computer system can determine the extent of nonuniform radiation attenuation over different parts of the body. From this, a nonuniform attenuation correction map of the body can be determined using well known methods and procedures. The nonuniform attenuation correction map is used to correct emission image data collected during emission studies.

Embodiments of the present invention are directed at improving the collection and use of transmission data for use in generating nonuniform attenuation correction factors to improve emission image data.

## A. DETECTOR SYSTEM

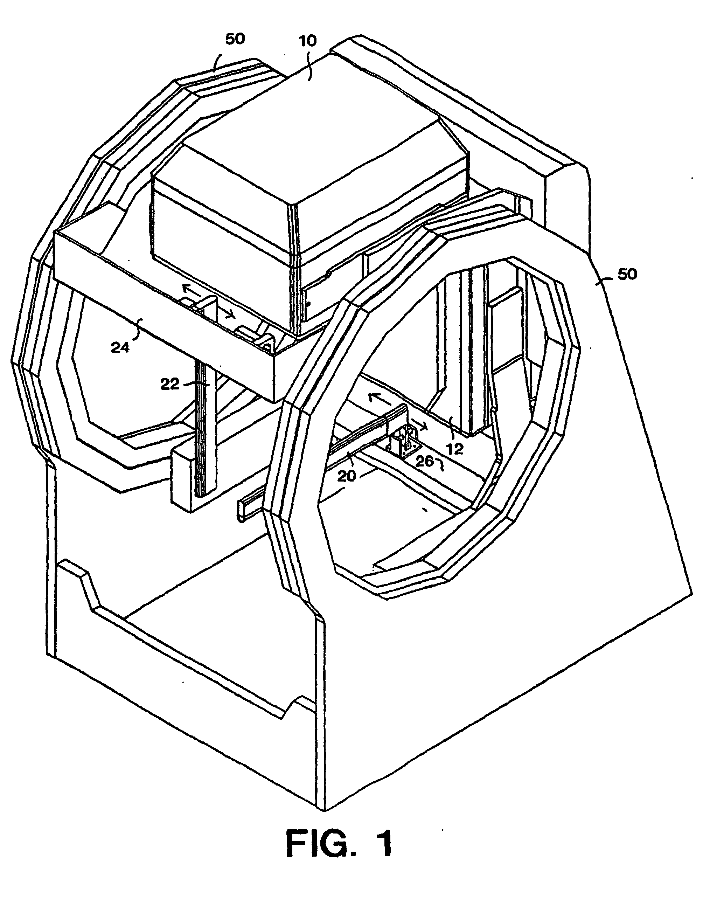

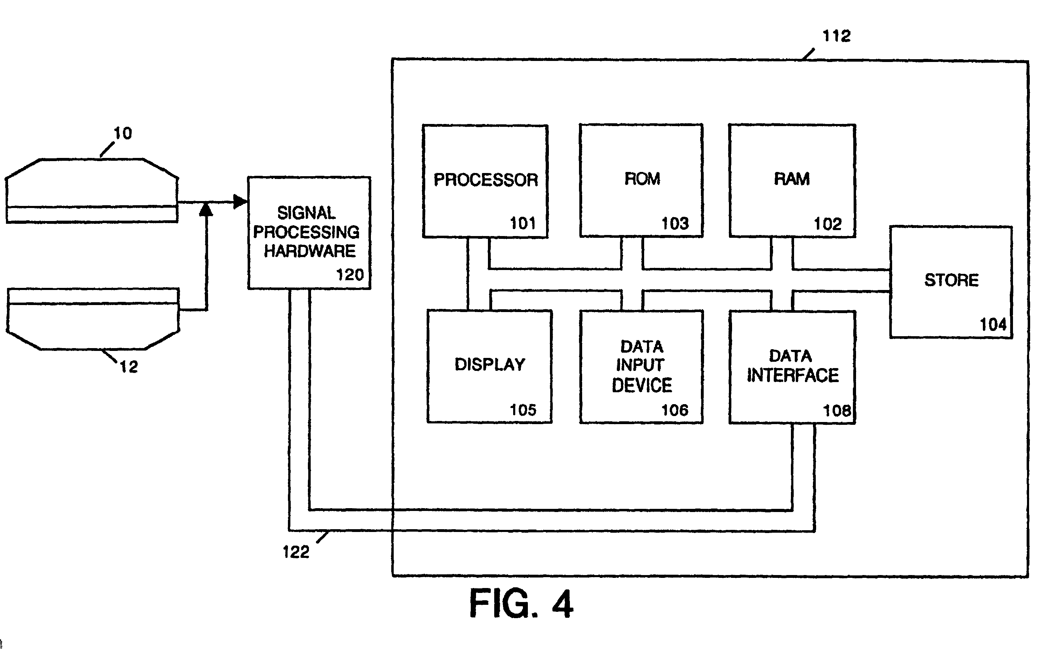

The detector system of an embodiment of the, present invention is illustrated in Figure 1. This is a dual head implementation, however, embodiments of the present invention can operate equally effective within a single or other multi- head embodiment. Regarding the dual head implementation, two scintillation detectors 10 and 12 are installed within the gantry (e.g., between gantry rings 50) and are rotatable about the center of the gantry ring 50. Each scintillation detector contains a crystal layer and an array of photomultiplier tubes, each PMT generates a separate channel signal responsive to light energy released by the crystal layer in response to a gamma interaction therein. As shown in Figure I, the detectors are at a 90 degree angle with respect to each other. A table (not shown) is placed into the gantry ring 50 and a patient rests on top of the table for imaging. Channel signals from the detectors are then sent to signal processing hardware 120 and to a computer system 112 for image processing (including corrections), see Figure 4.

The detectors 10 and 12 of Figure 1 can contain event detection circuitry that transforms the signals from the photomultipliers to digital signals representative of the spatial coordinate of each detected event and the event energy. This logic can also be externally located (for instance, see Figure 4, signal processing hardware 120). An event, or "count" is reported by the detectors to a computer system 112 for correction, analysis, storage and image generation. The detectors can collect and report radiation that is emitted from a patient (e.g., emission image data) and can also collect and report radiation emitted form a line source (transmission image data). Transmission data is utilized, among other things, for generation of attenuation correction distributions to compensate for nonuniform attenuation attributable to the patient (e.g., the chest region in cardiac studies).

A separate radiation emitting line source (with collimator) is mounted and associated with each scintillation detector. For instance, line source assembly 22 is associated with detector 12 and line source assembly 20 is associated with detector 10. Also, line source assembly 22 is mounted on rail 24 and the base of line source assembly 22 can move along the long axis of rail 24, as shown in order to displace ("scan") across the field of view of the associated detector. Likewise, line source assembly 20 is mounted on rail 26 and the base of line source assembly 20 can move along the long axis of rail 26, as shown in order to displace ("scan") across the field of view of the associated detector. It is appreciated that when the detectors rotate about the center of gantry ring 50, the associated emission line source will rotate in like form and degree. The line sources are utilized by the present invention for irradiating a patient in order to gather transmission data for the generation of the nonuniform attenuation correction map that is stored in memory for the patient. The nonuniform attenuation correction map is used to compensate for nonuniform attenuation of the emission data which is also collected by the present invention detectors 10 and 12.

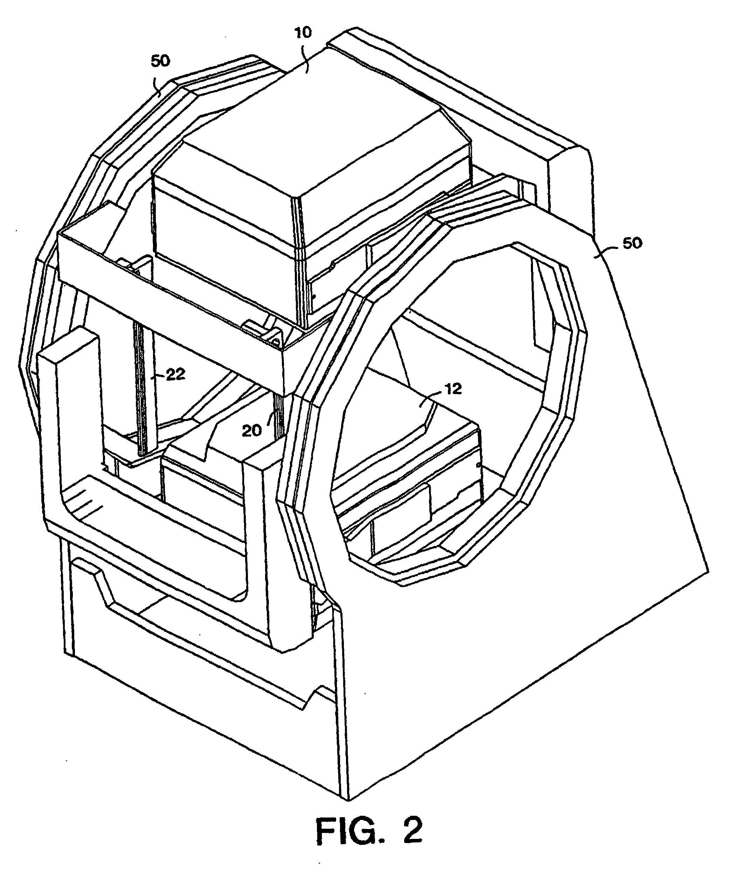

The scintillation detectors 10 and 12 can rotate about the gantry 50 so such that they are in the positions (180 degree orientation) shown in Figure 2. Figure 2 also illustrates the line source assemblies 22 and 20 in their storage position.

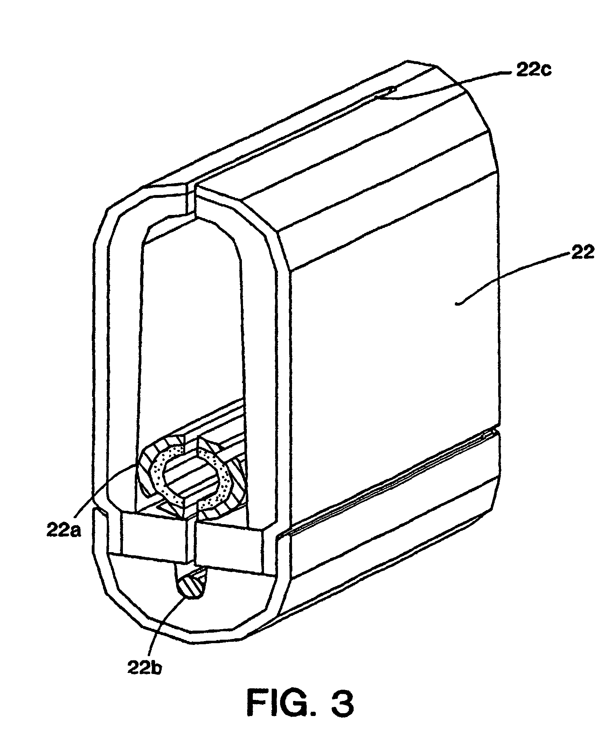

Figure 3 illustrates a cross section of an exemplary design of the scanning line source assembly 22 of one embodiment of the present invention in more detail (line source assembly 20 is similar). The radiation emitting source or rod is shown as 22b. The radiation is emitted through a small aperture and then through a shutter 22a that rotates to vary the amount of radiation emitted. Radiation is allowed pass through small openings places on either side of the shutter 22a. The radiation then exits via slot (collimator) 22c. Different types of line sources 22b can be utilized within the scope of the present invention, including a Tc-99m filled line source or a line source using Gd-153, Am241, or Co-57. Generally, the transmission source should be of a different photo peak energy than the emission source. As will be discussed in further herein, various embodiments of the present invention include special radiation filters within the line source assemblies.

## B. COMPUTER PROCESSOR SYSTEM

Refer to Figure 4 which illustrates components of a general purpose computer system 112 used by the present invention for processing image information supplied from gamma camera detectors 10 and 12. The general purpose computer system 112 is capable of performing image processing functions (e.g., processing emission and transmission data). The computer system 112 comprises an address/data bus 100 for communicating information within the system, a central processor 101 coupled with the bus 100 for executing instructions and processing information, a random access memory 102 coupled with the bus 100 for storing information and instructions for the central processor 101, a read only memory 103 coupled with the bus 100 for storing static information and instructions for the processor 101, a data storage device 104 such as a magnetic or optical disk and disk drive coupled with the bus 100 for storing image information and instructions, a display device 105 coupled to the bus 100 for displaying information to the computer user, an alphanumeric input device 106 including alphanumeric and function keys coupled to the bus 100 for communicating information and command selections to the central processor 101, a cursor control device (part of the data input device 106) coupled to the bus for communicating user input information and command selections to the central processor 101, and a communication device 108 coupled to the bus 100 for communicating command selections to the processor 101. A hardcopy device (e.g., printer) may also be coupled to bus 100.

The display device 105 of Figure 4 utilized with the computer system 112 of the present invention may be a liquid crystal device, cathode ray tube, or other display device suitable for creating graphic images and alphanumeric characters recognizable to the user. The cursor control device allows the computer user to dynamically signal the two dimensional movement of a visible symbol (pointer) on a display screen of the display device 105. Many implementations of the cursor control device are known in the art including a trackball, finger pad, mouse, joystick or special keys on the alphanumeric input device 105 capable of signaling movement of a given direction or manner of displacement.

The computer system 112 of Figure 4 interfaces with the gamma detector 10 and 12 via signal processing hardware circuits 120 over bus 122. The signal processing hardware 120 can compose amplification circuitry and analog to digital conversion circuits for converting channel signals from the detectors to digital data for transmission to the computer system 112. It is appreciated that channel signal information from the detectors 10 and 12 are converted into count density information by the computer system 112 and stored within the computer's memory 102 in matrix form, depending on the type of imaging session performed. This is true for both transmission and emission data. Nonuniform attenuation correction maps are stored in the computer memory 102 as well. The signal processing hardware 120 converts photomultiplier output into spatial coordinate data and event energy for detected events. Events within similar spatial coordinates are "binned" together in the memory 102 of the computer system in order to generate image information and form count density information.

This image information is collected in the form of a matrix of N rows by N columns. The size of the detector's effective field of view and the number of rows and columns of a particular matrix define the "size" of a pixel of the matrix. A pixel corresponds to one cell or "bin" of the matrix. Image matrices are generally collected at different ECT angles and then a reconstruction is done, using tomographic reconstruction to generate a three-dimensional image of an organ.

It is appreciated that the computer system 112 also controls movement of the detectors on the gantry ring 50 and also controls line source motion controllers for controlling the movement of the line sources 20 and 22.

Although the scintillation detectors 10, 12 are capable of reporting scintillation events with great accuracy, the computer system 112 collects and interprets the data depending on reference matrix sizes (e.g., 64 x 64, 128 x 128, 512 x 512 and 1024 x 1024). These sizes are programmable. Further, a given matrix size can be allocated to certain portions of the field of view of the detector. For instance, in a cardiac study, a 512 x 512 matrix can be allocated to only the region of the detectors' field of view that covers the heart. Therefore, when data is received from the scintillation detector regarding the energy and location of a detected interaction, this information is "binned" (e.g., placed) into the appropriate matrix entry that corresponds to the location of the interaction as reported by the detector. Count information reported by the detectors is binned into memory 102 and image data is taken from there. This is true for both transmission and emission data.

## II. SCAN SPEED EMBODIMENT

The present invention includes a scan speed embodiment wherein a minimum exposure time period is determined, patient by patient, for exposure to radiation emitted from the scanning line sources 20 and 22 for collection of transmission data. Patients vary by thickness and the more thick the patient, the more the patient needs to be irradiated to gather sufficient transmission data due to transmission radiation attenuation. However, the present invention determines the minimum dosage of radiation required to obtain sufficient transmission image data in order to reduce exposure time on a patient by patient basis. To promote the health of the patient, it is advantageous to reduce this exposure time to the patient. In order for the nonuniform attenuation correction map to be properly determined by the present invention (and there are a number of different ways in which this data can be computed) each elemental bin ("cell" or "pixel") of the image matrix for the patient must collect a certain minimum number of counts as a result of the transmission exposure. Using the reported transmission data, from a pre-scan phase, and knowing the original exposure of radiation, the gamma camera system of the present invention can determine a nonuniform attenuation correction map of the patient. Mechanisms for computing attenuation correction maps based on the above information are well known.

If there are more than the minimum number of counts per bin as a result of the transmission exposure, then the nonuniform attenuation correction distribution can still be properly computed, but the patient will be exposed to an unnecessary dosage of radiation to collect the transmission data. This embodiment of the present invention determines the particular dosage of radiation (e.g., exposure time) required in order to not unnecessarily expose the patient to transmission radiation.

Generally, in order to perform the above, the present invention provides two different transmission scan sequences or "phases." A first or "prescan" phase is performed and is a rapid single pass scan of low radiation dosage performed to obtain the minimum count density measured as a result of the prescan. Although a single pass scan is utilized for the prescan phase under the preferred embodiment of the present invention, multiple scan passes can also be contemplated within the scope of the present invention. The information of the prescan phase determines the duration of the normal transmission scan, which is the second transmission scan phase. The normal transmission scan is used to generate a nonuniform attenuation correction map. This process is explained in further detail below. It is appreciated that under the preferred embodiment of the present invention, the second or "normal" transmission scan phase is a multi-pass scan phase.

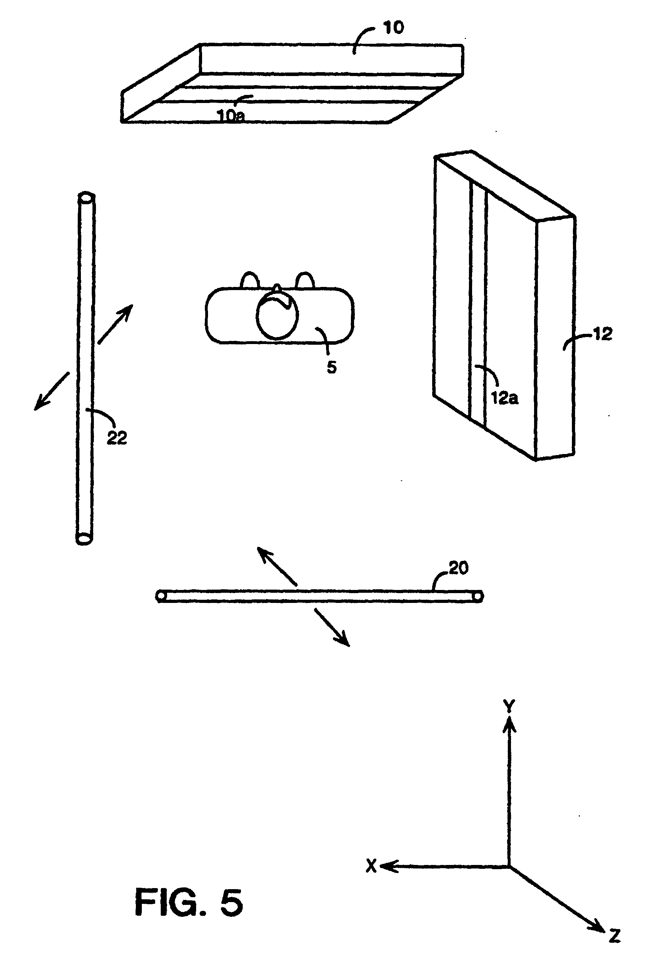

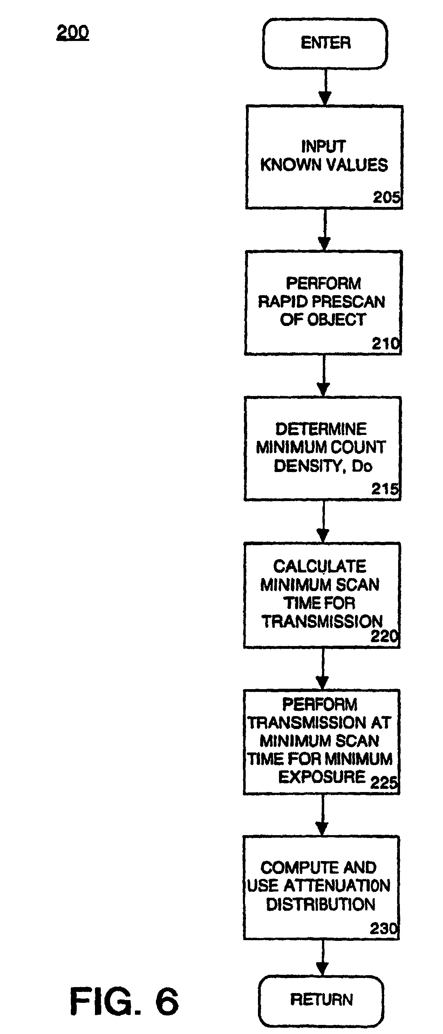

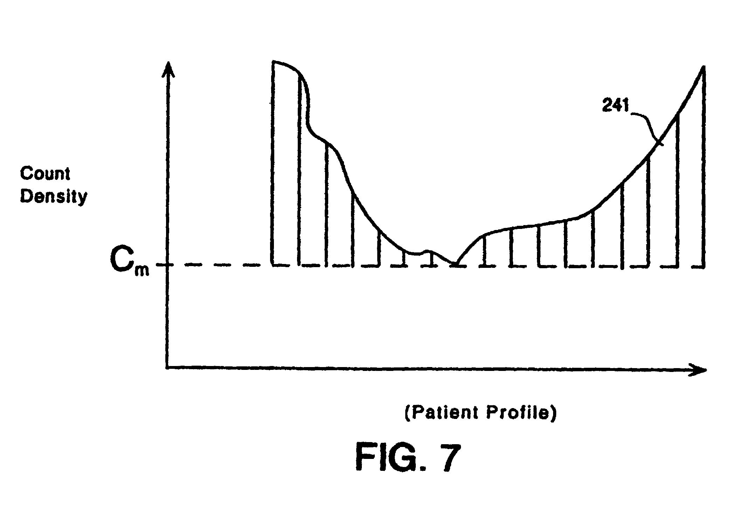

The processing 200 of this embodiment of the present invention is performed via general purpose computer system 112 and is illustrated with respect to Figure 6. Within the discussions of process 200, reference is also made to Figure 5 and Figure 7. The process 200 enters and at the first block 205, the known values are input from the data input device 106. The known values input at block 205 consist of (1) the minimum required counts per bin, Co, (which is also called the minimum count density) and (2) the time period for the prescan transmission radiation, Tp. The minimum required counts per bin, Co, in order to compute the nonuniform attenuation correction distribution will vary depending on the type of nonuniform attenuation correction map desired and the type of scan performed. Since this embodiment of the present invention can effectively operate to generate a number of different types of attenuation distributions based on a number of different types of scans, there is no specific minimum count number. However, with respect to the disclosed embodiment herein, the exemplary minimum number of counts is 20 to 30 (e.g., Co = 20-30).

Also, in one embodiment, the prescan exposure duration is programmable depending on the type of prescan performed and the type of nonuniform attenuation correction map desired. With respect to the disclosed exemplary embodiment, this value is in the range of 5-15 seconds (Tp = 5-15). It is appreciated that at block 205, the values Co and Tp can be programmed into computer system 112 as default values and therefore no data input is required unless these defaults are to be altered.

At block 205, the patient is placed onto a table and placed with the gantry ring 50. The scintillation detectors are arranged such that they will image different views of the patient. This can be performed a number of different ways and an exemplary arrangement is illustrated in Figure 5. As shown in Figure 5, the view is looking into the gantry ring structure 50 of the detector system (at the head of the patient 5 located along the Z axis). Scintillation detectors 10 and 12 are oriented at right angles to each other with detector 10 on top and detector 12 at the side. Line source assembly 20 scans the patient down the Z axis while the surface of detector 10 is above and within the XZ plane.

Transmission detection region 10a receives collimated radiation as the scanning line source assembly 20 travels down axis Z. Region 10a scans in synchronization with assembly 20. Similarly, line source assembly 22 scans the patient down the Z axis while the detector 12 is within the YZ plane. Transmission region 12a receives collimated radiation as the scanning line source assembly 22 travels down axis Z. Region 12a moves in synchronization with assembly 22. During the prescan operation, the time period required for the scanning line sources to move completely down the Z axis to radiate the patient 5 is the prescan time Tp. Therefore, the shorter the period selected, the faster the line sources move during the prescan and the longer the period, the slower the line sources move during the prescan. After the prescan, the two line sources 20 and 22 will again scan the patient 5 during the normal transmission scan.

Referring back to Figure 6, with the patient 5 placed in the gantry and the time period Tp selected, the present invention at block 210 prescans the patient, under computer system 112 control, by having both line sources 22 and 20 radiate the patient 5 while moving along the Z axis. Detectors 12 and 10 collect and report the transmission data via regions 10a and 12a to the computer system 112. When the prescan of block 210 is complete, the computer system stores a first count density information and then computer system 112 analyzes this transmission data at block 215 to determine the minimum count density measured, Cm, as a result of the transmission exposure.

Figure 7 illustrates an exemplary transmission map and shows count density versus the patient profile. Computer system 112 stores the "prescan" transmission map in memory 102. The map can also be stored in disk 104. Typically the side portion of the patient is the most attenuated portion and therefore contains the smallest count density measured, Cm. As the patient is thinner at the edges, the count density is larger at these parts of the profile. As the patient is thicker at the center, the count density is smaller at these regions. The result of the patient profile 241 is a U shaped "prescan" transmission map as shown in Figure 7. It is appreciated that a number of different methods and mechanisms are known in the art to compute a transmission map and different maps can be generated. For instance, a transmission map can be computed in three dimensional space (by gathering transmission data with ECT motion), the transmission map can be presented based on patient profile. Regardless of the way in which the "prescan" transmission data is presented, the present invention at block 215 determines the portion of the patient that most attenuates the transmission exposure. The count density of the "prescan" transmission map at this portion is the minimum count density measured by the prescan, Cm. At block 215, therefore, the "prescan" transmission map 241 is measured and the lowest measured count density, Cm, is recorded to memory 102.

At block 220, the present invention computes the minimum scan time required to perform the normal transmission scan that is used to generate the nonuniform attenuation correction factors of the present invention. The following relationship is utilized:

$$\frac{Tp}{Cm} = \frac{Ts}{Co}$$

Wherein the ratio between the time period of exposure for the prescan transmission exposure period (Tp) over the measured minimum count density for this prescan transmission (Cm) should be equal to the ration between the minimum time required to perform the normal transmission exposure (Ts) over the minimum count density required to perform the normal transmission exposure (Co). The values Co and Tp are input at block 205. The value Cm is computed at block 215. The above relationship can be rewritten as:

$$Ts = Co\ \frac{Tp}{Cm}$$

The computer system 112 at block 220 performs the above procedure in order to compute Ts, the minimum exposure time period required to insure that the minimum required count density, Co, is acquired by the normal transmission exposure scan time, Ts. At this time period, Ts, the part of the patient causing the most attenuation in the transmission data will still allow the collection of the minimum counts required of transmission data for generating a sufficient nonuniform attenuation correction map.

At block 225, the present invention computer system 112 directs the detector system to perform a normal transmission scan phase of the patient (similar to the scan performed in block 210), however, the normal transmission scan is performed using an exposure period of Ts. As discussed above, the second or "normal" transmission scan phase is a multi-pass scan phase across various angles of rotation around the patient. As a result of this normal transmission exposure, transmission data is collected by both detectors 10 and 12 at different projection angles and a normal transmission map (a second count density) is generated at block 230. From this, a nonuniform attenuation correction distribution is utilized for the correction of emission image data collected from the patient. The nonuniform attenuation correction map is stored in memory 102.

Utilizing the above procedure, the patient 5, is exposed to the minimum required radiation dosage during the normal transmission exposure. This is advantageous because of the varying thickness of different patients. If one exposure dosage was developed for all patients, smaller patients would be subjected to an unnecessarily long transmission exposure or larger patients would not have enough exposure to provide a usable attenuation correction map. Therefore, since the present invention determines the optimum exposure dosage on a patient by patient basis, each patient receives the minimum transmission dosage required to generate a sufficient nonuniform attenuation correction distribution.

The above procedure can be utilized with respect to a single transmission scan across a patient at a known or given angle relationship with respect to the detector and the patient. However, this technique is extended to include determining the minimum transmission exposure durations required (for each angle) within a transmission session involving ECT motion. This aspect of the present invention involves performing the prescan (of duration Tp) with both line source 20 and line source 22 in order to determine a minimum measured count value for both the anterior and lateral dimensions for a given object during the prescan. In other words, a Cm(anterior) and a Cm(lateral) can be obtained, one from each scintillation detector, during the prescan. By dividing the above count densities by the prescan Tp duration, a count rate can be determined for both the anterior and lateral dimensions. These count rates are expressed as Cr(anterior) and Cr(lateral).

$$Cr(anterior) = Cm(anterior)\ /Tp$$

$$Cr(lateral) = Cm(lateral)\ /Tp$$

Based on a body contour of the object (e.g., cross sectional profile of object) in conjunction with the lateral and anterior measured count rates, a particular transmission count rate can be determined by the computer system 112, using geometry, for each angle of rotation for an ECT scan. This count rate for a given angle is the transmission rate through the body for that given angle. This count rate value can be expressed as Cr(i), where (i) is the angle of rotation for a given ECT scan angle. The count rate for each angle, Cr(i), depends on the width and length of the object which can be supplied via the contour information. A number of well known techniques can be utilized to obtain body contour data.

Given the count rate, Cr(i), for each ECT angle, i, and given the minimum number of counts required for a given transmission scan, Co, the computer system 112 can compute the minimum time required for transmission at each ECT angle, Ts(i), according to the below relationship:

$$Ts(i) = Co\ /\ Cr(i)$$

The transmission duration, Ts(i), for each angle is then stored in the memory 102 of the computer system 112, for a given angle of rotation, i, the present invention will only expose the patient to the transmission radiation for a duration of Ts(i). This process continues until each angle of rotation is complete. Based on this information, a reconstruction is performed by the computer system 112 (using well known methods and procedures) in order to generate a three dimensional transmission map of the object. From this, a complete nonuniform attenuation correction map is derived for each angle of rotation. This embodiment of the present invention is particularly advantageous in reducing the transmission exposure of the patient because of the number of different transmission exposures (e.g., one for each angle or rotation).

It is appreciated that within the scope of the present invention, as an alternative operation to the above, a uniform scan rate can also be applied across all angles of ECT rotation.

One embodiment of the present invention using the above ECT technique, utilizes two detectors 10, 12 oriented at 90 degrees and uses both line sources 20, 22 to scan along the patient so that anterior and lateral transmission information are simultaneously gathered by each detector, respectively. In such case, the line sources radiate the patient simultaneously during prescan along the patient's long axis. Then the values Cm(lateral) and Cm(anterior) can be computed from the transmission maps of the detectors. As discussed above, using this information the minimum transmission duration can be obtained for each angle of rotation of the detector pair. The normal transmission scan for each angle can then be performed.

While the present invention has been described in particular embodiments, it should be appreciated that the present invention is construed according to the below claims.

## Claims

1. An apparatus for collecting transmission data associated with an object, said apparatus comprising:

    a radiation detector for detecting radiation and reporting image information;
    a source of radiation for emitting transmission radiation through said object to said radiation detector;
    a processor coupled to receive information from said radiation detector and coupled to control said source of radiation arranged to perform a first transmission prescan over said object for a first duration (Tp) and arranged to generate in response thereto a first count density information (Cm);
    said processor further being arranged to compute a second duration (Ts) for a second transmission scan phase as a function of said first count density information; and
    said processor further being arranged to control said source of radiation to perform said second transmission scan phase over said object for said second duration and to generate in response thereto a second count density information (Co), said second duration representing a minimum duration for object exposure required to generate said second count density information.

2. An apparatus as described in Claim 1 wherein said source of radiation is a scanning line source.

3. An apparatus as described in Claim 2 wherein said radiation detector is a scintillation detector of a gamma camera.

4. An apparatus as described in Claim 1 wherein said processor is for computing a set of attenuation correction factors based on said second count density information, said set of attenuation correction factors used for correcting image data gathered by emission scanning of said radiation detector.

5. An apparatus as described in Claim 1 wherein said second count density information is a distribution over different portions of said object and wherein said processor is for receiving, as input, a minimum count density wherein said distribution contains at least said minimum count density for each portion of said object.

6. An apparatus as described in Claim 5 wherein said processor is for analyzing said first count density information and determining a minimum observed count density in response thereto, wherein said minimum observed count density corresponds to a portion of said object exhibiting maximum attenuation.

7. An apparatus as described in Claim 6 wherein said processor determines said second duration according to the procedure:

$$Ts = Co \; \frac{Tp}{Cm}$$

wherein Ts is said second duration, Co is said minimum observed count density, Tp is said first duration, and Cm is said minimum count density of said first count density information.

8. A computer implemented method of collecting transmission data associated with an object, using a nuclear camera system having a scintillation detector for receiving radiation and reporting image information; a line source of radiation for emitting transmission radiation through an object to said scintillation detector; and a computer system, said method comprising the steps of:

    controlling said line source of radiation to perform a first transmission prescan over said object for a first predetermined duration (Tp) and generating in response thereto a first count density information (Cm);

computing a second duration (Ts) for a second transmission scan phase as a function of said first count density information; and

controlling said line source of radiation to perform said second transmission scan phase over said object for said second duration and generating in response thereto a second count density information (Co), said second duration representing a minimum duration of exposure of said object required to generate said second count density information.

9. A method as described in Claim 8 further comprising the steps of generating a set of attenuation correction factors based on said second count density information, said set of attenuation correction factors for use in correcting image data acquired during emission scanning.

10. A method as described in Claim 9 wherein said second count density information is a distribution over different portions of said object and further comprising the step of receiving as input a minimum count density wherein said distribution contains at least said minimum count density for each portion of said object.

11. A method as described in Claim 10 wherein said step of computing a second duration comprises the steps of:

analyzing said first count density information and determining a minimum observed count density in response thereto; and

computing said second duration according to the procedure:

$$Ts = Co \, \frac{Tp}{Cm}$$

wherein Ts is said second duration, Co is said minimum observed count density value, Tp is said first predetermined duration, and Cm is said minimum count density value.

12. A method as described in Claim 11 wherein said minimum observed count density corresponds to a portion of said object exhibiting maximum attenuation.

**Patentansprüche**

1. Eine Einrichtung zum Sammeln von Durchstrahlungsdaten, die einem Objekt zugeordnet sind, wobei die Einrichtung aufweist:

einen Strahlungsdetektor zum Erfassen der Strahlung und zum Übermitteln von Bildinformationen;

eine Strahlungsquelle zum Emittieren von Transmissionsstrahlung durch das Objekt auf den Strahlungsdetektor;

einen Prozessor, der so eingekoppelt ist, daß er Informationen aus dem Strahlungsdetektor empfängt und die Strahlungsquelle steuert, wobei die Anordnung so getroffen ist, daß sie eine erste Durchstrahlungsvorabtastung über das Objekt für eine erste Dauer (Tp) ausführt und in Abhängigkeit davon erste Impulsdichteinformationen (Cm) erzeugt;

wobei der Prozessor ferner so angeordnet ist, daß er eine zweite Dauer (Ts) für eine zweite Durchstrahlungsabtastphase als Funktion der ersten Impulsdichteinformationen berechnet; und

wobei der Prozessor ferner so angeordnet ist, daß er die Strahlungsquelle so steuert, daß die zweite Durchstrahlungsabtastphase über das Objekt für die zweite Dauer ausgeführt wird, und daß er in Abhängigkeit davon zweite Impulsdichteinformationen (Co) erzeugt, wobei die zweite Dauer eine minimale Dauer für eine Objektbestrahlung darstellt, die erforderlich ist, um die zweiten Impulsdichteinformationen zu erzeugen.

2. Eine Einrichtung nach Anspruch 1, wobei die Strahlungsquelle eine abtastende Linienquelle ist.

3. Eine Einrichtung nach Anspruch 2, wobei der Strahlungsdetektor ein Szintillationsdetektor einer Gammakamera ist.

4. Eine Einrichtung nach Anspruch 1, wobei der Prozessor der Berechnung einer Menge von Abschwächungskorrekturfaktoren auf der Grundlage der zweiten Impulsdichteinformationen dient, wobei die Menge der Abschwächungskorrekturfaktoren zum Korrigieren von Bilddaten, die durch Emissionsabtastung des Strahlungsdetektors gewonnen worden sind, verwendet werden.

5. Eine Einrichtung nach Anspruch 1, wobei die zweiten Impulsdichteinformationen eine Verteilung über verschiedene Abschnitte des Objekts sind und wobei der Prozessor als Eingabe eine minimale Impulsdichte empfängt, wobei die Verteilung zumindest die minimale Impulsdichte für jeden Abschnitt des Objekts enthält.

6. Eine Einrichtung nach Anspruch 5, wobei der Prozessor dem Analysieren der ersten Impulsdichteinformationen und dem Bestimmen einer minimalen beobachteten Impulsdichte in Abhängigkeit davon dient, wobei die minimale beobachtete Impulsdichte einem Abschnitt des Objekts entspricht, der eine maximale Abschwächung zeigt.

**7.** Eine Einrichtung nach Anspruch 6, wobei der Prozessor die zweite Dauer gemäß der Prozedur bestimmt:

$$Ts = Co \frac{Tp}{Cm}$$

wobei Ts die zweite Dauer ist, Co die minimale beobachtet Impulsdichte ist, Tp die erste Dauer ist und Cm die minimale Impulsdichte der ersten Impulsdichteinformationen ist.

**8.** Ein computer-implementiertes Verfahren des Sammeln von Durchstrahlungsdaten, die einem Objekt zugeordnet sind, unter Verwendung eines Nuklearkamerasystems mit einem Szintillationsdetektor zum Empfangen von Strahlung und Übermitteln von Bildinformationen; einer Linienstrahlungsquelle zum Emittieren von Transmissionsstrahlung durch ein Objekt auf den Szintillationsdetektor; und einem Computersystem, wobei das Verfahren die Schritte umfaßt:

Steuern der Linienstrahlungsquelle derart, daß sie eine erste Durchstrahlungsvorabtastung über das Objekt für eine erste vorgegebene Dauer (Tp) durchführt, und Erzeugen erster Impulsdichteinformationen (Cm) in Abhängigkeit davon,
Berechnen einer zweiten Dauer (Ts) für eine zweite Durchstrahlungsabtastphase als Funktion der ersten Impulsdichteinformationen, und Steuern der Linienstrahlungsquelle derart, daß sie die zweite Durchstrahlungsabtastphase über das Objekt für die zweite Dauer durchführt, und Erzeugen zweiter Impulsdichteinformationen (Co) in Abhängigkeit davon, wobei die zweite Dauer eine minimale Dauer der Bestrahlung des Objekts darstellt, die erforderlich ist, um die zweiten Impulsdichteinformationen zu erzeugen.

**9.** Ein Verfahren nach Anspruch 8, ferner umfassend den Schritt des Erzeugens einer Menge von Abschwächungskorrekturfaktoren auf der Grundlage der zweiten Impulsdichteinformationen, wobei die Menge der Abschwächungskorrekturfaktoren der Verwendung beim Korrigieren von Bilddaten, die während einer Emissionsabtastung gewonnen worden sind, dient.

**10.** Ein Verfahren nach Anspruch 9, wobei die zweiten Impulsdichteinformationen eine Verteilung über verschiedene Abschnitte des Objekts sind und ferner umfassend den Schritt des Empfangens einer minimalen Impulsdichte als Eingabe, wobei die Verteilung zumindest die minimale Impulsdichte für jeden Abschnitt des Objekts enthält.

**11.** Ein Verfahren nach Anspruch 10, wobei der Schritt des Berechnens einer zweiten Dauer die Schritte umfaßt:

Analysieren der ersten Impulsdichteinformationen und Bestimmen einer minimalen beobachteten Impulsdichte in Abhängigkeit davon; und Berechnen der zweiten Dauer gemäß der Prozedur:

$$Ts = Co \frac{Tp}{Cm}$$

wobei Ts die zweite Dauer ist, Co der minimale beobachtete Impulsdichtewert ist, Tp die erste vorgegebene Dauer ist und Cm der minimale Impulsdichtewert ist.

**12.** Ein Verfahren nach Anspruch 11, wobei die minimale beobachtete Impulsdichte einem Abschnitt des Objekts entspricht, der eine maximale Abschwächung zeigt.

**Revendications**

**1.** Appareil permettant de recueillir des données de transmission associées à un objet, ledit appareil comprenant:

un détecteur de rayonnement qui permet de détecter un rayonnement et de fournir des informations d'image;
une source de rayonnement qui permet d'émettre un rayonnement de transmission à travers ledit objet vers ledit détecteur de rayonnement;
un processeur couplé afin de recevoir les informations dudit détecteur de rayonnement et couplé afin de commander ladite source de rayonnement, organisé pour exécuter un premier pré-balayage de transmission au-dessus dudit objet pendant une première durée (Tp) et organisé afin de produire en réponse, des premières informations de densité de comptage (Cm);
ledit processeur étant organisé de plus afin de calculer une seconde durée (Ts) d'une seconde phase de balayage de transmission en fonction desdites premières informations de densité de comptage; et
ledit processeur étant organisé de plus afin de commander ladite source de rayonnement pour exécuter ladite seconde phase de balayage de transmission au-dessus dudit objet pendant ladite seconde durée et afin de produire en réponse, des secondes informations de densité de comptage (Co), ladite seconde durée représentant une durée minimum d'exposition de l'objet, requise afin de produire lesdites secondes informations de densité de comptage.

2. Appareil selon la revendication 1, dans lequel ladite source de rayonnement est une source de ligne de balayage.

3. Appareil selon la revendication 2, dans lequel ledit détecteur de rayonnement est un détecteur à scintillation d'une caméra gamma.

4. Appareil selon la revendication 1, dans lequel ledit processeur permet de calculer un ensemble de facteurs de correction d'atténuation sur la base desdites secondes informations de densité de comptage, ledit ensemble de facteurs de correction d'atténuation étant utilisé afin de corriger des données d'image recueillies par le balayage d'émission dudit détecteur de rayonnement.

5. Appareil selon la revendication 1, dans lequel lesdites secondes informations de densité de comptage représentent une distribution au-dessus des différentes parties dudit objet et dans lequel ledit processeur permet de recevoir, comme entrée, une densité minimum de comptage dans laquelle ladite distribution contient au moins ladite densité minimum de comptage de chaque partie dudit objet.

6. Appareil selon la revendication 5, dans lequel ledit processeur permet d'analyser lesdites premières informations de densité de comptage et de déterminer une densité de comptage observée minimum en réponse, dans lequel ladite densité de comptage observée minimum correspond à une partie dudit objet qui présente une atténuation maximum.

7. Appareil selon la revendication 6, dans lequel ledit processeur détermine ladite seconde durée selon la procédure:

$$Ts = Co\frac{Tp}{Cm}$$

dans laquelle Ts est ladite seconde durée, Co est ladite densité de comptage observée minimum, Tp est ladite première durée et Cm est ladite densité de comptage minimum desdites premières informations de densité de comptage.

8. Procédé mis en application avec un ordinateur qui permet de recueillir des données de transmission associées à un objet, utilisant un système de caméra nucléaire ayant un détecteur à scintillation qui permet de recevoir le rayonnement et de rapporter des informations d'image, une source de ligne de rayonnement qui permet d'émettre le rayonnement de transmission à travers un objet vers ledit détecteur à scintillation, et un système d'ordinateur, ledit procédé comprenant les étapes de:

commande de ladite source de ligne de rayonnement pour exécuter un premier pré-balayage de transmission au-dessus dudit objet pendant une première durée prédéterminée (Tp) et produisant en réponse des premières informations de densité de comptage (Cm); calcul d'une seconde durée (Ts) d'une seconde phase de balayage de transmission en fonction desdites premières informations de densité de comptage; et commande de ladite source de rayonnement pour exécuter ladite seconde phase de balayage de transmission au-dessus dudit objet pendant ladite seconde durée et afin de produire en réponse, des secondes informations de densité de comptage (Co), ladite seconde durée représentant une durée minimum d'exposition de l'objet, requise afin de produire lesdites secondes informations de densité de comptage.

9. Procédé selon la revendication 8, comprenant de plus les étapes de production d'un ensemble de facteurs de correction d'atténuation sur la base desdites secondes information de densité de comptage, ledit ensemble de facteurs de correction d'atténuation étant utilisé afin de corriger des données d'image acquises pendant le balayage d'émission.

10. Procédé selon la revendication 9, dans lequel lesdites secondes informations de densité de comptage représentent une distribution au-dessus des différentes parties dudit objet et comprenant de plus l'étape de réception, comme entrée, d'une densité minimum de comptage dans laquelle ladite distribution contient au moins ladite densité minimum de comptage de chaque partie dudit objet.

11. Procédé selon la revendication 10, dans lequel ladite étape de calcul d'une seconde durée comprend les étapes de:

analyse desdites premières informations de densité de comptage et de détermination d'une densité de comptage observée minimum en réponse; et calcul d'une seconde durée selon la procédure:

$$Ts = Co\frac{Tp}{Cm}$$

dans laquelle Ts est ladite seconde durée, Co est ladite densité de comptage observée minimum, Tp est ladite première durée prédéterminée et Cm est ladite valeur de densité de comptage minimum.

12. Procédé selon la revendication 11, dans lequel ladite densité de comptage observée minimum correspond à une partie dudit objet qui présente une atténuation maximum.

FIG. 1

FIG. 2

**FIG. 3**

FIG. 4

FIG. 5

200

```
        ┌──────────┐
        │  ENTER   │
        └──────────┘
              │
              ▼
   ┌────────────────────┐
   │       INPUT        │
   │   KNOWN VALUES     │
   │                205 │
   └────────────────────┘
              │
              ▼
   ┌────────────────────┐
   │      PERFORM       │
   │   RAPID PRESCAN    │
   │     OF OBJECT      │
   │                210 │
   └────────────────────┘
              │
              ▼
   ┌────────────────────┐
   │     DETERMINE      │
   │  MINIMUM COUNT     │
   │   DENSITY, Do      │
   │                215 │
   └────────────────────┘
              │
              ▼
   ┌────────────────────┐
   │     CALCULATE      │
   │   MINIMUM SCAN     │
   │     TIME FOR       │
   │   TRANSMISSION     │
   │                220 │
   └────────────────────┘
              │
              ▼
   ┌────────────────────┐
   │      PERFORM       │
   │  TRANSMISSION AT   │
   │   MINIMUM SCAN     │
   │  TIME FOR MINIMUM  │
   │   EXPOSURE     225 │
   └────────────────────┘
              │
              ▼
   ┌────────────────────┐
   │    COMPUTE AND     │
   │  USE ATTENUATION   │
   │   DISTRIBUTION     │
   │                230 │
   └────────────────────┘
              │
              ▼
        ┌──────────┐
        │  RETURN  │
        └──────────┘
```

# FIG. 6

**FIG. 7**